# EUROPEAN PATENT SPECIFICATION

(11) **EP 1 530 045 B1**
(45) Date of publication and mention of the grant of the patent: **05.03.2008**
(21) Application number: 03025724.0
(22) Date of filing: 10.11.2003
(51) Int. Cl.: G01N 33/52, G01N 33/92

(54) **Reagent-containing membranes and laminates and methods of their preparation**
Reagenz-Membrane und -Laminate und Verfahren zur deren Herstellung
Membranes et structures stratifiées contenant des réactifs et méthodes de leur préparation

(30) Priority: 04.11.2003 US 517596
(43) Date of publication of application: 11.05.2005
(73) Proprietor: CHOLESTECH CORPORATION, Hayward, CA 94545-3808 (US)
(72) Inventor: Jones, Ronald, M., Mountain View, CA 94040 (US)
(74) Representative: Hess, Peter K. G.

(56) References cited:
- EP-A- 0 740 157
- EP-A- 1 357 383
- WO-A-96/15453
- US-A- 5 213 964
- US-A- 5 426 030
- US-A1- 2003 166 291

## Description

### Field of the Invention

The present invention relates to a method of impregnating a membrane or membrane laminate with a uniform and controlled amount of reagent. In particular aspects, the invention relates to preparing membrane laminates containing impregnated reagents, where the reagents are limited to a single respective layer of the laminate, and to such reagent-containing membranes and laminates and assay devices containing them.

### Background of the Invention

Diagnostic assays employing a strip format, where a porous material such as a fibrous strip or membrane is impregnated with diagnostic reagents, are in common use due to convenience, speed and reduced need for manipulation of reagents by the user. Uniform and reproducible application of reagents is important in the production of such devices, to ensure consistency and accuracy of test results.

Various methods have been employed for applying reagents to such test strips. For example, a porous membrane can be impregnated with a reagent simply by contacting the membrane with a solution of the reagent. To achieve uniform impregnation, a saturating amount of solution is typically used. However, this approach often produces non-uniform distribution of reagents. In particular, it is unsatisfactory for laminated membranes, since it generally does not limit dispersion of the reagent to a single layer, particularly if the layers are of similar materials. Spray coating or syringe coating of reagent to a surface of a membrane or laminate can provide better control of the amount of reagent applied, thus reducing the risk of dispersion to underlying layers in a laminate. However, coating by these methods also tends to be non-uniform, producing concentration gradients with more reagent in the center of the application area.

As described in the EP 1 329 724 A2 and the DE 102 04 606, another approach to applying reagents to different layers in a laminate is to coat or impregnate the layers separately, followed by lamination. The above documents disclose a high-density lipoprotein (HDL) assay device containing a reagent pad and an HDL-reaction membrane. Said reagent pad is produced by dispensing aqueous solution containing dextrane sulfate onto an asymmetric membrane in a certain dispensing rate. Using the same process, the HDL-reaction membrane is impregnated with an aqueous formulation. To complete the above assay device, the two impregnated membranes are separately or simultaneously attached by ultrasonic welding.

Document EP 0 740 157 A2 discloses to use a gravure coating procedure to produce labeled ligand coatings over a particulate layer. The idea of the EP'157 is to provide a coating that can be coated directly over the particulate layer, for example cloth, without an intervening barrier layer. This document does not relate to an impregnation of a membrane of a membrane laminate, i.e. to produce a laminate containing a first membrane impregnated with a first reagent, wherein said first reagent does not contact the second membrane in said laminate.

Document EP 1 357 383 relates to an assay device and a method for measuring the concentration of HDL associated with cholesterol in a blood fluid sample. The EP'383 utilizes two polymeric membranes, wherein the appropriate reagents are impregnated and the membranes are afterwards processed as a two-membrane layer for incorporation into the assay device. Thus, the two polymeric membranes are impregnated separately.

It is, however, disadvantageous that such an ultrasonic lamination or a thermal lamination may be unsuitable for heat sensitive reagents. Further, the use of an adhesive between layers may reduce the need for heating, but it leads to possible contamination of assay chemistry. It is also important to retain porosity of porous membranes, and any of these techniques can be detrimental in this respect.

It is therefore the technical problem of the present invention to provide a method for uniform application of reagents membranes and in particular to separate membranes in a laminate, without cross contamination between layers, and without exposing the reagents to excessive heat. Ideally, such a process would uniformly apply a controlled volume of reagent to one or both sides of such a laminate. It is a further problem of the present invention to provide an assay device comprising uniformly loaded membranes for determining reliable test results.

### Summary of the Invention

In one aspect, the invention provides a method of applying a reagent to a membrane in a membrane laminate, comprising first and second laminated membranes. The method comprises:
(a) providing a laminate of the first and second membranes, having an outer first membrane surface, a laminated first membrane surface, a laminated second membrane surface, and an outer second membrane surface;
(b) applying a controlled volume of a solution of a first reagent to the outer first membrane surface, by contacting the surface with a support having a surface containing a pattern, and
(c) drying the first membrane in the laminate,
to produce a laminate containing the first membrane impregnated with the first reagent, wherein the first reagent does not contact the second membrane in the laminate.

Preferably, the method applies said solution by means of a pattern comprising recesses filled with said solution, or by means of a pattern comprising projections applying said solution. Furthermore, said support preferably comprises a planar or cylindrical shape.

Preferably, the method further comprises impregnating the second layer with a second reagent, by (d) applying a controlled volume of a solution of the second reagent to the outer second membrane surface, by contacting the surface with a solid support containing recesses filled with the solution, and (e) drying the second membrane in the laminates, to produce a laminate containing the second membrane impregnated with the second reagent, wherein the second reagent does not contact the first membrane in the laminate.

Typically, the solid support is a rotating gravure cylinder, having a plurality of recesses or cells formed in the surface, as discussed further below. Preferably, said applying occurs simultaneously across the entire width of the first membrane outer surface, to produce a uniform application across the width of the membrane.

The "controlled volume" of solution is generally no more than a saturating amount of the solution, with respect to the absorptive capacity of the respective membrane, and is typically a subsaturating amount, as defined below. In certain embodiments, a given (first or second) reagent does not contact the laminated surface of the respective membrane in the finished laminate; that is, it penetrates only partially through the depth of the respective membrane. In other embodiments, a given (first or second) reagent contacts the laminated surface of the respective membrane in the finished laminate; that is, it impregnates the full thickness of the respective membrane, but it does not contact the other membrane in the laminate.

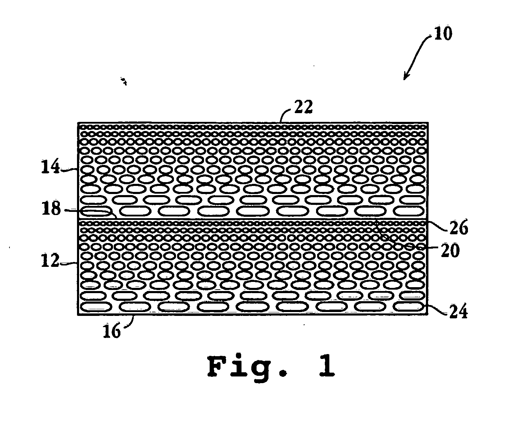

In one embodiment, at least the first membrane is an asymmetric membrane, i.e. having a small pored surface and a large pored surface. The second membrane may also be an asymmetric membrane. In selected embodiments, the outer first membrane surface is the large pored surface of the first membrane. In a further embodiment, the outer second membrane surface is the small pored surface of the second membrane. Such an embodiment is illustrated in Fig. 1, which shows a laminate **10** of first membrane **12** and second layer **14**, having outer first membrane surface **16**, laminated first membrane surface **18**, laminated second membrane surface **20**, and outer second membrane surface **22**. The laminated first membrane surface and laminated second membrane surface jointly form a laminate interface.

At least one of the reagents may be a heat sensitive reagent, such that laminating a membrane containing said reagent to a further membrane would produce a detrimental change in the reagent. In one embodiment, the first reagent comprises reagents effective to selectively remove non-HDL lipoproteins from a blood fluid sample passing through the first membrane, and the second reagent comprises reagents effective to produce an optically detectable signal proportional to a quantity of HDL-associated cholesterol in the second membrane.

In a related aspect, the invention provides a method of applying a reagent to a membrane, by applying a controlled volume of a solution of said reagent to a first surface of the membrane, by contacting the surface with a planar or cylindrical support having a surface containing a pattern of recesses filled with the solution. Again, the support is typically a rotating gravure cylinder. Further, the techniques and apparatus are preferably used which are described for applying reagents to a membrane in a membrane laminate.

The controlled amount of reagent may be a subsaturating amount. In one embodiment, following application of reagent and, preferably, drying, the reagent does not contact the opposite surface of said membrane; that is, it penetrates only partially through the depth of the membrane. The application of reagent preferably occurs simultaneously across the entire width of the first surface of the membrane. Accordingly, the concentration of the reagent is preferably uniform across the length and width of said membrane.

The membrane may be an asymmetric membrane, having a small pored surface and a large pored surface; in one embodiment, the first surface, to which reagent is applied, is the large pored surface. In other embodiments, the first surface is the small pored surface. In one preferred embodiment, the reagent comprises reagents effective to selectively remove non-HDL lipoproteins from a blood fluid sample passing through the membrane; alternatively, the reagent includes reagents effective to selectively precipitate non-HDL (LDL and VLDL) lipoprotein particles in the sample, and the precipitated LDL and VLDL particles are prevented by filtration from passing through the membrane. In this embodiment, when the membrane is an asymmetric membrane, the reagent is applied to the large pored surface. In another preferred embodiment, the reagent comprises reagents effective to produce an optically detectable signal proportional to a quantity of HDL-associated cholesterol in the membrane. In this embodiment, when the membrane is an asymmetric membrane, the reagent is applied to the small pored surface. In another aspect, the invention provides a membrane laminate for use in a diagnostic assay, comprising first and second laminated membranes which are joined at a laminate interface, wherein the first membrane in said laminate is impregnated with a first reagent, the second membrane is impregnated with a second reagent, and the reagent in at least one of the membranes is absent in a laminar region of that membrane adjacent the laminate interface, such that the reagents in the two membranes are physically separated by at least that laminar region. Preferably, the concentration of each reagent or reagent system is uniform across the length and width of the respective membrane.

In selected embodiments, at least one said reagent is heat sensitive, such that laminating a membrane containing said reagent to a further membrane would produce a detrimental change in the reagent.

Preferably, at least the first membrane is an asymmetric membrane, having a small pored surface and a large pored surface. In selected embodiments, the small pored surface faces the laminate interface. The second membrane may also be an asymmetric membrane; in selected embodiments, its large pored surface faces the laminate interface.

In a preferred embodiment, the first reagent comprises reagents effective to selectively remove non-HDL lipoproteins from a blood fluid sample passing through said first membrane; alternatively, the first reagent includes reagents effective to selectively precipitate non-HDL (LDL and VLDL) lipoprotein particles in the sample, and the precipitated LDL and VLDL particles are prevented by filtration from passing through the laminate interface. In these embodiments, the second reagent comprises reagents effective to produce an optically detectable signal proportional to a quantity of HDL-associated cholesterol in the second membrane. Preferably, in such a laminate, the reagent in the second membrane is absent in a laminar region of that membrane adjacent the laminate interface.

In a related aspect, the invention may be used to provide an assay device for assaying HDL in a blood or serum sample, comprising
(a) a substrate having a sample-receiving well for receiving an aliquot of the sample,
(b) a membrane laminate comprising first and second laminated membranes which are joined at a laminate interface, wherein
   (i) the first membrane is impregnated with reagents effective to selectively precipitate LDL and VLDL lipoprotein particles in the sample, and the precipitated LDL and VLDL particles are prevented by filtration from passing through the laminate interface;
   (ii) the second membrane is impregnated with reagents effective to produce an optically detectable signal proportional to a quantity of HDL-associated cholesterol in the second membrane; and
   (iii) the reagent in at least one of the membranes, and preferably the second membrane, is absent in a laminar region of that membrane adjacent the laminate interface, such that the reagents in the two membranes are physically separated by at least that laminar region.

In the laminate, as described above, the concentration of each reagent is preferably uniform across the length and width of the membrane. The device also comprises (c) a filter between the sample-receiving well and the first membrane of the laminate, for removing red blood cells in the sample as the sample migrates from the sample-receiving well to the laminate, wherein the well, filter and laminate are or can be placed in fluid communication with each other.

These and other objects and features of the invention will become more fully apparent when the following detailed description of the invention is read in conjunction with the accompanying drawings.

### Brief Description of the Drawings

Figure 1 is a cross section view of two laminated asymmetric membranes to which reagents may be applied in accordance with one embodiment of the invention;
Figure 2 is a schematic diagram showing a coating system for application of reagents to a laminate, in accordance with one embodiment of the invention; and
Figure 3 is a side view of an assay device incorporating a membrane laminate, in accordance with one embodiment of the invention.

### Detailed Description of the Invention

### I. Reagent Application Method

The invention provides methods of impregnating or coating a laminate of at least two membranes with at least one reagent, such that each reagent is confined to its respective membrane. Preferably, the laminate includes at least two membranes, each containing a different reagent or reagent system.

One example of such a laminate is a membrane assembly used in a device designed for measuring the concentration of HDL-associated cholesterol in a blood sample which also containing LDL and VLDL particles. See, for example, Jones et al., US Appn. Pubn. No. 20030166291 and US Appn. Serial No. 10/410,671. However, the invention is useful for any application employing a laminate as described above, having reagerits impregnated in at least one, and preferably two, membrane layers, where each reagent must be confined to its respective layer.

The exemplary HDL assay laminate, as described further below, includes an HDL test membrane, in which HDL concentration is measured, and a reagent membrane, containing a binding and/or precipitation reagent, such that the membrane is effective to selectively remove non-HDL lipoproteins from the fluid sample, before the sample contacts the test membrane.

Preferably, each of the membranes in the laminate is a porous asymmetric membrane; that is, a membrane having a pore size gradient across its thickness. An exemplary two-membrane layer **10** comprising two asymmetric membranes **12** and **14** is shown in cross section in Fig. 1, with the preferred orientation shown, with larger pores at **24** and smaller pores at **26** in membrane **12**.

In order for the HDL assay to function accurately, the HDL test reagent must be restricted to it respective layer. If the HDL reagent is present in the reagent layer, the color developed in the assay reaction will generally represent some fraction of non-HDL cholesterol in addition to the analyte, HDL-associated cholesterol.

The applicants have found that a gravure printing process, in which fluid is applied to a substrate from recesses in a solid plate having planar or arbitrary shape, or, typically, a rotating cylinder, allows controlled-volume deposition of different reagents to opposite surfaces of a laminated membrane. By controlling the deposition volume, each reagent can be limited to its respective layer in the laminate.

According to a further preferred embodiment of the present invention, the reagents are applied to separate membranes or membrane laminates by a letterpress technique. To this end, the support has a planar or arbitrary suitable configuration, preferably the configuration of a cylinder. Said support comprises a pattern of projections applying the reagents to the membrane. Preferably, said reagents are provided having a consistency to temporarily adhere to said projections so that they are applied in a controlled manner.

In gravure coating, a network of depressions, typically closely spaced cells, is etched on the surface of a metal cylinder. The cells are loaded with fluid, and the rotating cylinder transfers the fluid to a substrate. The cells have a defined volume, thus limiting the amount of liquid that can be transferred to the substrate. Typically, a cylinder has a volume of about 30-50 µl per square inch of cylinder surface (about 4.5 - 8 µl per cm²). (In this sense "cylinder surface" assumes a smooth surface, corresponding to the surface area of the substrate contacted by the cylinder. It does not include the additional surface area provided by the recesses themselves.) Other volume capacities can also be used. The recesses are generally uniformly sized and closely spaced, such that, upon transfer of liquid drops to the membrane, they coalesce into a uniform layer of liquid.

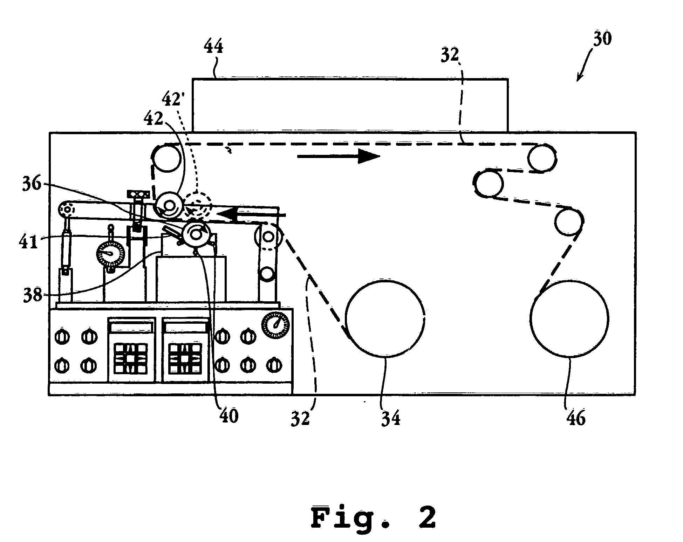

A gravure coating system that may be used in carrying out the processes described herein is shown at **30** in Fig. 2. (As used herein, "coating" a membrane includes applying a reagent such that it penetrates beyond the surface and may impregnate the entire thickness of the membrane.)

The laminated membrane or "web" **32** is fed from a roll **34**. The gravure cylinder **36** is typically copper or copper-plated steel or aluminum; a thin layer of chrome is often applied for durability. As described above, the surface of the cylinder is etched with a plurality of small cells effective to hold a defined quantity of liquid. A cylinder is chosen with a pattern of wells that is effective to apply a uniform coating with the given coating system. The cylinder may be partially submerged in a bath or tray of the fluid to be applied. Alternatively, as preferred for the instant method, a reagent chamber **38** may be used to deliver reagent solution to the cylinder via conduits **40**. The chamber reduces exposure of the reagents to the atmosphere.

As the cylinder turns, the excess solution is wiped off the cylinder by a flexible doctor blade **41** which contacts the cylinder between the reagent chamber or tray and the membrane **32**. Solution remaining in the recessed cells is then transferred to the desired surface of the membrane.

In one embodiment, the membrane **32** is held in tangential contact (i.e., contacting a few degrees of the circumference of the cylinder) with the gravure cylinder by a takeup cylinder **42,** a process referred to as "kiss" gravure. Alternatively, the web may pass between the gravure cylinder **36** and an impression or "backup" cylinder, as shown at **42**'. In this process, takeup cylinder **42** is generally absent. In an offset gravure process (not shown), fluid is transferred from the gravure cylinder to a rubber coated transfer roll, which then applies the solution to the substrate.

The substrate is then passed through a dryer **44,** preferably an IR dryer blower, and taken up on takeup roll **46.** The other surface of the laminate is then coated in a similar manner.

The gravure coating process may be carried out in a direct mode, where the cylinder rotates in the same direction as the membrane feed. Alternatively, in reverse gravure, as illustrated in Fig. 2, the rotational direction of the cylinder is opposite to the travel direction of the membrane. This arrangement results in a shearing force being applied to the solution as it is transferred to the substrate, which can result in a smoother coating.

The volume of reagent solution applied to the membrane as it contacts the gravure roll is limited by the volume of the cells on the cylinder surface, as noted above. The amount actually transferred from the cells to the membrane depends on additional factors, such as cylinder speed, roll pressure, web speed, and solution components. Accordingly, the amount transferred to the membrane can be further controlled by proper selection of the system configuration, contact time between the membrane and cylinder (determined by web speed and direction relative to gravure rotation speed and direction) and surfactant level. Surfactant is included to facilitate transfer of the solution to the membrane surface, which might otherwise repel an aqueous solution, depending on the hydrophobicity of the membrane material.

For asymmetric membranes, the penetration of reagent into the membranes is also dependent on pore size and distribution. For example, with reference to Fig. 1, solution applied to surface **16** (large pored) will be drawn into the membrane by capillary action to a significantly greater extent than solution applied to surface **22** (small pored).

Accordingly, application of a controlled volume of reagent solution to one side of a membrane on the outer surface of a laminate, followed by drying, produces a laminate in which the reagent does not penetrate beyond that membrane. The controlled amount may be a subsaturating amount; that is, a volume of the solution which is less than that which would, if applied to a selected membrane, penetrate the entire membrane by capillary flow. In some cases, a saturating or near-saturating volume may be used.

In accordance with the invention, an amount of reagent solution is applied to the membrane to give the desired amount of penetration into the thickness or depth of the membrane. By simultaneously applying the controlled amount of solution across the width of the membrane, the application method also provides a uniform concentration of reagent across the length and width of the membrane. By "uniform" is meant that there are no significant concentration gradients or changes across the length or width of the membrane. This uniformity is effective to give consistent assay readings for sample presented to different points on the surface of the laminate. Preferably, following application of reagent and drying, the amount of reagent present at different points on the surface of a membrane in the laminate, or at different points on a given laminar surface parallel to this surface, varies by no more than about 5%.

The application method is useful for heat sensitive reagents, where lamination of a membrane containing the reagent, under normal thermal lamination conditions, would produce a detrimental change in the reagent and/or its function in an assay carried out on the laminate. Such a change could include a change in the morphology or distribution of the reagent as well as chemical changes in the reagent.

The application method may also be advantageously used to apply a controlled amount of reagent in a uniform manner to a single membrane, by contacting the surface of the membrane with a planar or cylindrical support having a surface containing a pattern of recesses filled with the solution. Again, the support is typically a rotating gravure cylinder, as described above. As in laminate coating, the application of reagent by this method preferably occurs simultaneously across the entire width of the first surface of the membrane; accordingly, the concentration of the reagent is substantially uniform across the length and width of the membrane.

The controlled amount of reagent may be a subsaturating amount. In one embodiment, following application of reagent and, preferably, drying, the reagent does not contact the opposite surface of the membrane; that is, it penetrates only partially through the depth of the membrane.

The method is advantageous even when a saturating amount is to be applied, in that it produces a membrane in which reagents are impregnated more uniformly, as compared to prior art methods such as submersion in a saturating amount of solution or spray coating.

The invention also provides membranes impregnated with reagents in accordance with this method. Of particular interest are membranes for use in diagnostic assays.

The membrane may be an asymmetric membrane, having a small pored surface and a large pored surface; the reagent may be applied to either surface. In one preferred embodiment, the reagent comprises reagents effective to selectively remove non-HDL lipoproteins from a blood fluid sample passing through the membrane; alternatively, the reagent includes reagents effective to selectively precipitate non-HDL (LDL and VLDL) lipoprotein particles in the sample, and the precipitated LDL and VLDL particles are prevented by filtration from passing through the membrane. In these embodiments, when the membrane is an asymmetric membrane, the reagent is preferably applied to the large pored surface. In another preferred embodiment, the reagent comprises reagents effective to produce an optically detectable signal proportional to a quantity of HDL-associated cholesterol in the membrane. In this embodiment, when the membrane is an asymmetric membrane, the reagent is preferably applied to the small pored surface.

### II. Membrane Laminates

In another aspect, the invention provides laminated membranes having reagents impregnated in at least one, and preferably two, membrane layers, where each reagent (or reagent system) is confined to its respective layer. Generally, different layers contain different reagent systems. Preferably, the reagent is applied to a laminated membrane in accordance with the methods described above. Of particular interest are membrane laminates for use in diagnostic assays.

Such a laminate comprises first and second laminated membranes, impregnated with first and second reagents, respectively, which are joined at a laminate interface. At least one of the first and second reagents is absent in a laminar region of the respective membrane adjacent the laminate interface, such that the reagents in the two membranes are physically separated by at least that laminar region.

Preferably, the concentration of each said reagent is uniform across the length and width of the respective membrane containing that reagent. This uniformity is effective to give consistent assay readings for sample presented to different points on the surface of the laminate. Preferably, the amount of reagent present at different points on the surface of a membrane in the laminate, or at different points on a given laminar surface parallel to this surface, varies by no more than about 5%.

In one embodiment, the laminate is one designed for use in determining the level of HDL-associated cholesterol in a fluid sample. See, for example, Jones et al., US Appn. Pubn. No. 20030166291 and US Appn. Serial No. 10/410,671, cited above. In this case, the first membrane contains reagents effective to selectively remove non-HDL lipoproteins from a blood fluid sample passing through the membrane. The non-HDL lipoproteins may be removed, for example, by selective binding to a reagent contained or immobilized within the membrane. Alternatively or in addition, these components are removed by selective precipitation by the reagent and are prevented by filtration from passing through the laminate interface into the second membrane.

Such reagents include polyanionic compounds, such as sulfonated polysaccharides, heparin, or phosphotungstate, in combination with a group II cation, such as Mg²⁺, Mn²⁺, or Ca²⁺. A preferred reagent is a sulfonated polysaccharide, such as dextran sulfate, having a typical molecular weight of 50-500 KDa, in combination with magnesium acetate or chloride, optionally buffered to maintain neutral pH. The reagents may also be immobilized to the membrane.

The HDL test membrane contains reagents for quantification of HDL-associated cholesterol, as is known in the art. See, for example, co-owned U.S. Patent Nos. 5,213,964, 5,213,965, 5,316,196 and 5,451,370. Typically, the assay reagents include cholesterol esterase, for releasing free cholesterol from HDL, cholesterol oxidase, for producing H₂O₂ by reaction with free cholesterol, peroxidase, and a coupled dye system which is converted, in the presence of peroxidase and H₂O₂, to a distinctively colored signal reaction product. Assay reagents are known in the art for quantification of other blood components, e.g. total cholesterol, triglycerides, or glucose, and frequently comprise similar enzyme/coupled dye systems.

Preferably, at least the first membrane is an asymmetric membrane, having a small pored surface and a large pored surface. The small pored surface preferably faces the laminate interface, such that the large pored surface is on the outer surface of the laminate. This orientation allows free access of sample into the membrane through the larger pores, and prevents passage of any precipitated or other solid material through the smaller pores. The second membrane may also be an asymmetric membrane, preferably having its large pored surface facing the laminate interface, and the small pored surface on the other surface of the laminate. This orientation presents the more uniform surface of the membrane for optical scanning and quantitation of assay results.

The preparation of asymmetric membranes is described, for example, in U.S. Patent Nos. 4,629,563, 5,171,445, 5,886,059, 5,536,408, 5,562,826, and 4,774,192; in D. R. Lloyd, "Materials Science of Synthetic Membranes", ACS Symposium 269: 1-21 (1985). They are commercially available in a variety of pore sizes and pore size ratios. Materials of fabrication include polysulfones, polyethersulfones, polyamides, polyether amides, polyurethanes, cellulose acetate, polyvinyl pyrrolidone, polystyrenes and modified polystyrenes, as well as blends, copolymers, and laminar composites. For further description of preferred membrane types see e.g. US Appn. Pubn. No. 20030166291 and US Appn. Serial No. 10/410,671, cited above. An exemplary asymmetric membrane is a polysulfone or polyethersulfone membrane, such as BTS 83 membranes provided by Pall Corporation (San Diego, CA).

In the exemplary HDL assay laminate, each membrane typically has a thickness of about 100-150 µm and an absorption capacity of about 80 µl/sq in (about 12.4 µl/cm²). Minimum pore sizes typically range from 0.01 to 10 µm, with maximum/minimum pore size ratios up to 100 or more.

In one embodiment, the laminate includes at least one reagent which is heat sensitive, such that laminating a membrane containing the reagent to a further membrane produces a detrimental change in the reagent. The detrimental change could include any or all or a change in morphology, distribution or the actual chemical structure of the reagent. Such laminates are advantageously prepared by the reagent application methods described herein.

### III. Assay Devices

Membrane laminates for use in diagnostic assays, such as the HDL assay laminate described herein, are typically incorporated into an assay device for convenient use. The invention accordingly may be used to provide assay devices containing membrane laminates as described herein. For example, an assay device for assaying HDL in a blood or serum sample comprises:
(a) a substrate having a sample-receiving well for receiving an aliquot of a blood fluid sample,
(b) a membrane laminate comprising first and second laminated membranes which are joined at a laminate interface, and
(c) a filter between the sample-receiving well and the first membrane of the laminate, for removing red blood cells in the sample, as the sample migrates from the sample-receiving well to the laminate. The membrane laminate of (b) is a laminate as described above, where the first membrane is impregnated with reagents effective to selectively precipitate non-HDL (LDL and VLDL) lipoprotein particles in said sample, and the precipitated LDL and VLDL particles are prevented by filtration from passing through the laminate interface; the second membrane is impregnated with reagents effective to produce an optically detectable signal proportional to a quantity of HDL-associated cholesterol in said second membrane; and the reagent in at least one of these membranes is absent in a laminar region of that membrane adjacent the laminate interface, such that the reagents in the two membranes are physically separated by at least that laminar region. It is particularly preferred that the HDL assay reagents are absent in a laminar region of the second membrane adjacent the laminate interface.

Preferably, the concentration of each reagent is uniform across the length and width of the membrane containing the reagent, as described above. The membranes are preferably asymmetric membranes, oriented as described above, where the outer surface of the reagent membrane is the large pored (dull) surface and the outer surface of the assay membrane is the small pored (shiny) surface.

The sample well, filter and laminate of (a)-(c) above are or can be placed in fluid communication with each other. For example, these elements may reside on a common substrate, or they may reside on one or more separate substrates and be brought into fluid communication during the course of the assay. Different embodiments of such an assay device include those described in Jones et al., US Appn. Pubn. No. 20030166291 and US Appn. Serial No. 10/410,671, cited above.

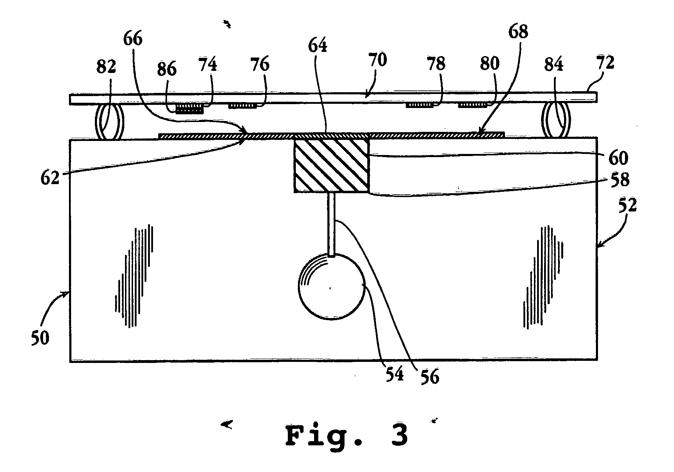

One embodiment is shown in Fig. 3. The apparatus **50** includes a main body or support **52** which defines the sample well **54.** The well is in fluid contact with a sieving pad **60**, which may be carried in a notched region **58** formed in the upper edge of the support. The fluid contact may be direct, or as in the device shown in Fig. 3, provided by a capillary conduit **56** formed in the plate at the base of the well. The support is preferably a plastic plate, with the well, notched region and/or capillary formed by standard molding or machining methods.

Sieving pad **60** carried in region **58** functions to partially remove large particulate matter (including blood cells) as the sample migrates through the pad matrix in a bottom-to-top direction as shown in the figure. Pad **60** is preferably formed of a glass fibrous matrix of material designed to draw aqueous fluid by surface wetting, and to retard the movement of blood cells as the blood sample is drawn through the matrix. One exemplary pad is a glass fiber filter, such as a GF/D or PD008 filter supplied by Whatman. The pad is dimensioned to absorb a defined volume of sample fluid, e.g. about 15-25 µl. Sieving pad **60** may additionally contain red blood cell capture reagents, such as lectins, antibodies specific for red blood cell surface membrane proteins, thrombin, or ion exchange agents.

The sieving pad, **60**, in turn, contacts an elongate strip or sample distribution matrix **62** which extends along the upper edge of plate **52**. This strip may also be supported by foam cushions or other supports. Matrix **62** serves to distribute sample fluid from a central sample-application region **64**, which is in fluid contact with pad **60**, to sample-collection regions such as **66, 68** within the matrix. The matrix is preferably formed of glass fibers. The packing density and thickness of the matrix are such as to absorb and distribute volumes of sample fluid, *e.g.*, 10-25 µl, supplied to the sample-application region of the strip to the sample-collection regions of the strip. One exemplary strip material is a F-165-25A glass fiber filter available from Whatman, having a packing density of about 0.2 gm/cm³ and a thickness of about 0.12 mm.

Device **50** also includes a reaction bar **70** composed of an elongate support **72,** and one or more multiple wettable, absorbent reaction test strips, shown as **74**, **76, 78** and **80**, carried on the lower surface of the support, as shown. Elements **74** and **86** form a membrane laminate as described herein, where **74** is the HDL assay membrane and **86** is the reagent membrane.

Support **72** is transparent or has windows or openings which allow the pads to be viewed through the support. Each test pad used in a particular assay contains analyte-dependent reagents effective to produce an analyte-dependent change in the pad which can be detected in a known manner.

The reaction bar is mounted on support **52** by mounting means effective to (a) maintain the device in a sample-distribution position, wherein the test membrane/reagent membrane laminate is spaced apart from the sample distribution matrix, and to (b) transfer the device to a test position, where the test membrane/reagent membrane laminate and the sample distribution matrix are in fluid communication. The mounting means can also be used to break such fluid communication after a desired amount of sample has entered the test pads, and/or after a determined contact time, by transferring the device from the test position to a position in which the test strip(s) are not in fluid communication with the sample distribution matrix (which may be the same as the "sample-distribution" position). Such transferring can be controlled by monitoring the reflectance at the top surface of the test pad, which reflects extent of wetting, as described in co-owned U.S. Patent No. 5,114,350. Alternatively, when the absorption capacity and rate of sample uptake of the pad material are known, the quantity of sample can be controlled with sufficient accuracy by using a predetermined contact time.

The mounting means can include, for example, a pair of resilient members, such as elastomeric blocks **82, 84**, which act to bias the test membrane/reagent membrane laminate toward a non-transfer or sample-distribution position, at which the laminate is spaced apart from the sample distribution matrix. By compression or release of the resilient members, fluid communication between sample distribution matrix **62** and the laminate can be selectively established and separated. The fluid communication may be via direct contact or through an intermediate element. The support blocks could be compressed by means of springs or a piston-like action. Alternatively, external mechanical devices could engage the main body **52** and/or support **62** and move one towards the other. An exemplary system is the Cholestech LDX® Analyzer, a self-contained, automated analyzer advantageous for use with assay devices such as described herein.

### EXAMPLES

Exemplary HDL assay laminates were prepared by gravure coating, as disclosed herein, and HDL assays on known standards were run using the laminates. The data showed excellent precision, demonstrating the effectiveness of the disclosed process for applying controlled and uniform quantities of the assay reagents.

The membrane laminate employed was a laminate of two BTS-83 polysulfone asymmetric membranes, laminated with the small pored (shiny) side of one membrane contacting the large pored (dull) side of the second membrane, prepared by Pall Corporation, San Diego CA. Widths of up to about 5 inches were used for coating.

Standard precipitation and HDL assay reagent mixtures were used. The precipitation solution included 3.9 mg/ml dextran sulfate (500,000 MW) and 7.9 mM Mg(OAc)₂ in water. A surfactant (Pluronic L64, produced by BASF, at a level of 0.05%) was also added to facilitate uptake onto the membrane.. The HDL assay reagent solution contained 80.3 U/ml cholesterol oxidase, 473 U/ml cholesterol esterase, 440 U/ml horseradish peroxidase, 4.14 mg/ml 4-aminoantipyrine, and 26.5 mg/ml TOOS (3-[ethyl(3-methylphenyl)amino]-2-hydroxy propanesulfonic acid) in water, with 3 mg/ml CHAPS added as surfactant.

A flexographic press with gravure cylinder was used to apply a constant volume of reagent solution to a rubber roller which was in contact with the membrane laminate (offset gravure process). The cylinder used for this experiment was a 65 line/inch, 30 BCM roll, where BCM refers to billion cubic microns, or microliters, per square inch of surface.

The precipitation reagents were first applied to the dull side of the first membrane.

Following application, the membrane was dried with an IR dryer blower. The HDL colorimetric assay reagents were then applied to the shiny side of the second membrane of the laminate, in a similar manner, and dried with an IR dryer blower.

The following HDL assay data was from laminated membranes prepared as described above, cut into 0.22 inch sections and assembled into assay cassettes, such as described in US Appn. Pubn. No. 20030166291 and US Appn. Serial No. 10/410,671, cited above. Assays were carried out according to standard procedures. The samples (eight HDL standards) were analyzed in an LDX^{®} analyzer, and the mean of eight cassettes was taken for each standard. Excellent precision and correlation with the standard values were obtained.

| HDL standard (mg/dL) | LDX^{®} mean result |
|---|---|
| 32.6 | 33.9 |
| 42.5 | 40.8 |
| 76.2 | 75.5 |
| 62.3 | 58.9 |
| 53.2 | 52.4 |
| 45.7 | 47.7 |
| 49.5 | 53.0 |

In the offset gravure process used for the above experiment, up to four passes of reagent solution were applied to each side of the membrane. Coat weight for each pass was determined by the loss in weight of the coating solution, and good consistency was observed from one pass to another.

A direct gravure process was found to apply the solution more efficiently than the offset process, generally making repeated passes unnecessary for this system. In a preferred process, the gravure cylinder spins in a direction opposite to the web feed direction (i.e., reverse gravure, as shown in Fig. 2), and the web contacts the cylinder without an impression cylinder ("kiss" gravure, as shown at **42** in Fig. 2). A cylinder is chosen with a pattern of wells that is effective to apply a uniform coating with the given coating system.

Membranes were coated using the reverse/kiss gravure process, with web speeds of 5-10 ft/min and cylinder rotation speeds of about twice the web speed for the precipitation reagent coating and about the same as the web speed for the HDL assay reagent coating. The quantity of solution applied to the membrane in these runs was generally about 65% of the saturating volume for the precipitation reagents, and about 45% of the saturating volume for the HDL assay reagents.

## Claims

1. A method of applying a reagent to a membrane (12, 14) in a membrane laminate (10), the method comprising:
(a) providing a laminate (10) of first (12) and second membranes (14), said laminate (10) having an outer first membrane surface (16), a laminated first membrane surface (18), a laminated second membrane surface (20), and an outer second membrane surface (22),
(b) applying a controlled volume of a solution of a first reagent to said outer first membrane surface (16), by contacting said surface (16) with a planar or cylindrical support (36) having a surface containing a pattern of recesses filled with said solution, and
(c) drying said first membrane (12) in said laminate (10),
to produce a laminate (10) containing said first membrane (12) impregnated with said first reagent, wherein said first reagent does not contact the second membrane (14) in said laminate (10).

2. The method according to claim 1, wherein said pattern comprises projections applying said solution.

3. The method of claim 1, wherein said controlled amount is a subsaturating amount.

4. The method of claim 1, further comprising:
(d) applying a controlled volume of a solution of said second reagent to said outer second membrane surface (22), by contacting said surface (22) with a solid support (36) containing recesses filled with said solution, and
(e) drying said second membrane (14) in said laminate (10), to produce a laminate (10) containing said second membrane (14) impregnated with said second reagent, wherein said second reagent does not contact the first membrane (12) in said laminate (10).

5. The method of claim 4, wherein said controlled amount of (d) is a subsaturating amount.

6. The method of claim 5, wherein said second reagent does not contact the laminated second membrane surface (20).

7. The method of claim 1, wherein at least said first membrane (12) is an asymmetric membrane, having a small pored surface and a large pored surface.

8. The method of claim 7, wherein said outer first membrane surface (16) is the large pored surface.

9. The method of claim 7, wherein said second membrane (14) is an asymmetric membrane, having a small pored surface and a large pored surface.

10. The method of claim 9, wherein said outer second membrane surface (22) is the small pored surface.

11. The method of claim 1, wherein said support (36) is a rotating gravure cylinder.

12. The method of claim 1, wherein said first reagent comprises reagents effective to selectively remove non-HDL lipoproteins from a blood fluid sample passing through said first membrane (12).

13. The method of claim 1, wherein said first reagent includes reagents effective to selectively precipitate non-HDL lipoprotein particles from a blood fluid sample, whereby the precipitated particles are prevented by filtration from passing through the laminated first membrane surface (18).

14. The method of claim 13, wherein said second reagent comprises reagents effective to produce an optically detectable signal proportional to a quantity of HDL-associated cholesterol in said second membrane (14).

15. The method of claim 1, wherein said applying occurs simultaneously across the entire width of the first membrane outer surface (16).

16. The method of claim 1, wherein said first reagent is heat sensitive, such that laminating a membrane (12) containing said reagent to a further membrane (14) produces a detrimental change in the reagent.

17. The method of claim 4, wherein said second reagent is heat sensitive.

## Patentansprüche

1. Ein Verfahren zum Auftragen eines Reagenz auf einer Membran (12, 14) in einer Schichtstruktur aus Membranen (10), wobei das Verfahren umfasst:
(a) Bereitstellen einer Schichtstruktur (10) aus ersten (12) und zweiten Membranen (14), wobei die Schichtstruktur (10) eine äußere erste Membranoberfläche (16), eine geschichtete erste Membranoberfläche (18), eine geschichtete zweite Membranoberfläche (20) und eine äußere zweite Membranoberfläche (22) aufweist;
(b) Auftragen einer bestimmten Menge einer Lösung eines ersten Reagenz auf die äußere erste Membranoberfläche (16) durch Kontaktieren dieser Oberfläche (16) mit einem ebenen oder zylindrischen Träger (36), welcher eine Oberfläche aufweist, die ein Muster von Vertiefungen enthält, welche mit der Lösung gefüllt sind; und
(c) Trocknen der ersten Membran (12) in dieser Schichtstruktur (10),
um eine Schichtstruktur (10) herzustellen, die die erste Membran (12) umfasst, welche mit dem ersten Reagenz imprägniert ist, wobei das erste Reagenz die zweite Membran (14) in der Schichtstruktur (10) nicht kontaktiert.

2. Das Verfahren gemäß Anspruch 1, wobei das Muster Vorsprünge aufweist, welche die Lösung auftragen.

3. Das Verfahren gemäß Anspruch 1, wobei diese bestimmte Menge eine nicht saturierende Menge ist.

4. Das Verfahren gemäß Anspruch 1, weiter umfassend:
(d) Auftragen einer bestimmten Menge einer Lösung des zweiten Reagenz auf die äußere zweite Membranoberfläche (22) durch Kontaktieren dieser Oberfläche (22) mit einem festen Träger (36), welcher Vertiefungen enthält, die mit der Lösung gefüllt sind; und
e) Trocknen der zweiten Membran (14) in der Schichtstruktur (10), um eine Schichtstruktur (10) herzustellen, welche die zweite Membran (14) enthält, die mit dem zweiten Reagenz imprägniert ist, wobei das zweite Reagenz die erste Membran (12) in der Schichtstruktur (10) nicht kontaktiert.

5. Das Verfahren gemäß Anspruch 4, wobei diese bestimmte Menge in (d) eine nicht saturierende Menge ist.

6. Das Verfahren gemäß Anspruch 5, wobei das zweite Reagenz die beschichtete zweite Membranoberfläche (20) nicht kontaktiert.

7. Das Verfahren gemäß Anspruch 1, wobei mindestens die erste Membran (12) eine asymmetrische Membran ist, die eine Oberfläche mit kleinen Poren und eine Oberfläche mit großen Poren aufweist.

8. Das Verfahren gemäß Anspruch 7, wobei die äußere erste Membranoberfläche (16) die Oberfläche mit großen Poren ist.

9. Das Verfahren gemäß Anspruch 7, wobei die zweite Membran (14) eine asymmetrische Membran ist, die eine Oberfläche mit kleinen Poren und eine Oberfläche mit großen Poren aufweist.

10. Das Verfahren gemäß Anspruch 9, wobei die äußere zweite Membranoberfläche (22) die Oberfläche mit kleinen Poren ist.

11. Das Verfahren gemäß Anspruch 1, wobei der Träger (36) eine rotierende Gravurwalze ist

12. Das Verfahren gemäß Anspruch 1, wobei das erste Reagenz Reagenzien umfasst, die wirksam sind, um selektiv Nicht-HDL-Lipoproteine aus einer Blutflüssigkeit-Probe zu entfernen, welche durch die erste Membran (12) läuft.

13. Das Verfahren gemäß Anspruch 1, wobei das erste Reagenz Reagenzien umfasst, die wirksam sind, um Nicht-HDL-Lipoprotein-Teilchen aus einer Blutflüssigkeit-Probe auszufällen, wobei die ausgefällten Teilchen durch Filtrieren daran gehindert werden, durch die geschichtete erste Membranoberfläche (18) hindurchzutreten.

14. Das Verfahren gemäß Anspruch 13, wobei das zweite Reagenz Reagenzien umfasst, die wirksam sind, um ein optisch detektierbares Signal zu produzieren, das proportional zu einer Menge von HDL-assoziiertem Cholsterin in der zweiten Membran (14) ist.

15. Das Verfahren gemäß Anspruch 1, wobei das Auftragen gleichzeitig über die gesamte Breite der äußeren Oberfläche (16) der ersten Membran stattfindet.

16. Das Verfahren gemäß Anspruch 1, wobei das erste Reagenz wärmeempfindlich ist, so dass das Beschichten einer Membran (12), welche das Reagenz enthält, mit einer anderen Membran (14) eine nachteilige Änderung in dem Reagenz bewirkt.

17. Das Verfahren gemäß Anspruch 4, wobei das zweite Reagenz wärmeempfindlich ist.

## Revendications

1. Un procédé pour appliquer un réactif sur une membrane (12, 14) dans une stratifié de membranes (10), le procédé comprenant :
a) l'obtention d'un stratifié (10) d'une première (12) et d'une seconde membrane (14), ce stratifié (10 ayant une surface extérieure de première membrane (16), une surface stratifiée de première membrane (18), une surface stratifiée de seconde membrane (20) et une surface extérieure de seconde membrane (22),
b) l'application d'un volume contrôlé d'une solution d'un premier réactif sur la surface extérieure de première membrane (16), par mise en contact de cette surface (16) avec un support plan ou cylindrique (36) possédant une surface contenant un motif d'évidements remplis de ladite solution, et
c) le séchage de ladite première membrane (12) dudit stratifié (10),
pour produire un stratifié (10) contenant ladite première membrane (12) imprégnée dudit premier réactif, où ledit premier réactif ne vient pas en contact avec la seconde membrane (14) dudit stratifié (10).

2. Le procédé de la revendication 1, dans lequel ledit motif comprend des projections d'application de ladite solution.

3. Le procédé de la revendication 1, dans lequel ladite quantité contrôlée est une quantité subsaturante.

4. Le procédé de la revendication 1, comprenant en outre :
d) l'application d'un volume contrôlé d'une solution dudit second réactif sur ladite surface extérieure de seconde membrane (22), par mise en contact de cette surface (22) avec un support solide (36) contenant des évidements remplis de ladite solution, et
e) le séchage de ladite seconde membrane (14) dudit stratifié (10) pour produire un stratifié (10) contenant ladite seconde membrane (14) imprégnée par ledit second réactif, où ledit second réactif ne vient pas en contact avec la première membrane (12) dudit stratifié (10).

5. Le procédé de la revendication 4, dans lequel ladite quantité contrôlée de (d)) est une quantité subsaturante.

6. Le procédé de la revendication 5, dans lequel ledit second réactif ne vient pas en contact avec la surface stratifiée de la seconde membrane (20).

7. Le procédé de la revendication 1, dans lequel au moins ladite première membrane (12) est une membrane asymétrique, ayant une surface à petits pores et une surface à grands pores.

8. Le procédé de la revendication 7, dans lequel ladite surface extérieure de première membrane (16) est la surface à larges pores.

9. Le procédé de la revendication 7, dans lequel ladite seconde membrane (14) est une membrane asymétrique, ayant une surface à petits pores et une surface à larges pores.

10. Le procédé de la revendication 9, dans lequel ladite surface extérieure de seconde membrane (22) est la surface à petits pores.

11. Le procédé de la revendication 1, dans lequel ledit support (36) est un cylindre de gravure rotatif.

12. Le procédé de la revendication 1, dans lequel ledit premier réactif comprend des réactifs efficaces pour sélectivement éliminer les lipoprotéines non-HDL à partir d'un échantillon de fluide sanguin traversant ladite première membrane (12).

13. Le procédé de la revendication 1, dans lequel ledit premier réactif comprend des réactifs efficaces pour sélectivement précipiter des particules de lipoprotéines non-HDL à partir d'un échantillon de fluide sanguin, de manière à empêcher les particules précipitées, par filtration, de traverser la surface stratifiée de première membrane (18).

14. Le procédé de la revendication 13, dans lequel ledit second réactif comprend des réactifs efficaces pour produire un signal optiquement détectable proportionnel à une quantité de cholestérol associée à HDL dans ladite seconde membrane (14).

15. Le procédé de la revendication 1, dans lequel ladite application intervient simultanément sur toute l'étendue de la largeur de la surface extérieure de la première membrane (16).

16. Le procédé de la revendication 1, dans lequel ledit premier réactif est sensible à la chaleur, de sorte que la stratification d'une membrane (12) contenant ledit réactif sur une autre membrane (14) produise une modification préjudiciable du réactif.

17. Le procédé de la revendication 4, dans lequel ledit second réactif est sensible à la chaleur.
